# EUROPÄISCHE PATENTANMELDUNG

(11) **EP 0 654 280 A1**
(43) Veröffentlichungstag der Anmeldung: **24.05.1995**
(21) Anmeldenummer: 94108018.6
(22) Anmeldetag: 25.05.1994
(51) Int. Cl.: A61M 5/315

(54) **Einwegspritze**

(30) Priorität: 19.11.1993 DE 4339528
(71) Anmelder: Firma Carl Freudenberg, D-69469 Weinheim (DE)
(72) Erfinder: Schumacher, Herbert, D-69517 Gorxheimertal (DE); Osen, Ernst, Dr., D-69488 Birkenau (DE); Messner, Norbert, Dr., D-35279 Neustadt (DE); Ecknig, Dirk, D-68163 Mannheim (DE)

(57) **Zusammenfassung**

Einwegspritze zur Verabreichung flüssiger Wirkstoffe (1) an lebende Menschen oder Tiere, umfassend einen mit dem Wirkstoff (1) gefüllten Hohlzylinder (2), auf den eine Injektionsnadel (3) aufsetzbar ist und der einen Kolben (4) umschließt, der mittels einer Kolbenstange (5) in Richtung der Injektionsnadel (3) bewegbar ist. Der Kolben (4) ist aus einer thermoplastisch verarbeitbaren Kautschukmischung erzeugt und in Richtung des Wirkstoffs (1) durch eine den Hohlzylinder (2) berührende, umlaufende und in radialer Richtung einfederbare Dichtlippe (6) begrenzt.

## Beschreibung

Die Erfindung betrifft eine Einwegspritze zur Verabreichung flüssiger Wirkstoffe an lebende Menschen oder Tiere, umfassend einen mit dem Wirkstoff gefüllten Hohlzylinder, auf den eine Injektionsnadel aufsetzbar ist und der einen Kolben umschließt, der mittels einer Kolbenstange in Richtung der Injektionsnadel bewegbar ist.

Derartige Einwegspritzen sind allgemein bekannt, wobei der Kolben und die Kolbenstange materialeinheitlich und einstückig ineinander übergehend ausgebildet sind. Die Kolbenstange und der Kolben bestehen beispielsweise aus Polyethylen oder Nylon. Die Dichtwirkung zwischen dem Kolben und dem Hohlzylinder, der ebenfalls bevorzugt aus Polyethylen besteht, ist wenig befriedigend. Selbst unter Einhaltung sehr geringer Herstellungstoleranzen ist der Anpreßdruck des Kolbens an die Innenwandung des Hohlzylinders in Teilbereichen, die einander in Umfangsrichtung benachbart zugeordnet sind, unterschiedlich groß, wobei die Gefahr einer dadurch bedingten Leckage durch Relaxationserscheinungen des Kolbens zusätzlich vergrößert wird. Der Kolben, der zylinderförmig ausgebildet ist und auf der dem Wirkstoff zugewandten Seite eine ebene, sich in radialer Richtung erstreckende Stirnwand aufweist, verhindert außerdem eine weitgehend vollständige Entleerung des Hohlzylinders.

Der Erfindung liegt die Aufgabe zugrunde, eine Einwegspritze der vorgenannten Art derart weiterzuentwickeln, daß die obengenannten Nachteile zuverlässig vermieden werden und die Einwegspritze diesbezüglich verbesserte Gebrauchseigenschaften aufweist. Insbesondere soll die Abdichtung zwischen dem Kolben und dem Hohlzylinder derart verbessert werden, daß Undichtigkeiten in diesem Bereich vermieden werden und der Wirkstoff nahezu vollständig aus dem Hohlzylinder entleert werden kann.

Diese Aufgabe wird erfindungsgemäß mit den kennzeichnenden Merkmalen von Anspruch 1 gelöst. Auf vorteilhafte Ausgestaltungen nehmen die Unteransprüche bezug.

Im Rahmen der vorliegenden Erfindung ist es vorgesehen, daß der Kolben aus einer thermoplastisch verarbeitbaren Kautschukmischung erzeugt und in Richtung des Wirkstoffs durch eine den Hohlzylinder berührende, umlaufende und in radialer Richtung einfederbare Dichtlippe begrenzt ist. Der Kolben ist als Dichtelement ausgebildet und aus elastomerem Werkstoff hergestellt, so daß die in radialer Richtung elastisch nachgiebige Dichtlippe mit der Innenfläche des Hohlzylinders dichtend in Eingriff ist. Die Dichtlippe berührt den Hohlzylinder innenseitig entlang einer Umfangslinie mit übereinstimmender radialer Vorspannung. Herstellungsbedingte Toleranzen des Hohlzylinders und/oder des Kolbens können dadurch problemlos ausgeglichen werden, so daß eine Zuverlässige Abdichtung bedingt ist. Gelangen beispielsweise in Bewegungsrichtung der Kolbenstange mehrere funktionstechnisch hintereinander geschaltete Dichtlippen zur Anwendung ist eine erhöhte Sicherheit gegen einen Wirkstoffverlust gegeben. Ist demgegenüber nur eine den Hohlzylinder berührende, umlaufende und in radialer Richtung einfederbare Dichtlippe vorgesehen, ist die Reibung zwischen dem Kolben und dem Hohlzylinder auf ein Minimum begrenzt und eine kräftearme, ruckfreie Betätigung der Kolbenstange sichergestellt, so daß der zu verabreichende Wirkstoff einfach und exakt dosiert werden kann.

Gemäß einer vorteilhaften Ausgestaltung kann der Kolben mittels eines Innengewindes an einem Außengewinde der Kolbenstange festgelegt sein, wobei das Innengewinde in Bewegungsrichtung des Kolbens bevorzugt kürzer bemessen ist als das Außengewinde. Die Kolbenstange ist bevorzugt derart ausgebildet, daß sie auf der dem Wirkstoff abgewandten Seite mit einer tellerförmigen Druckplatte versehen ist, wobei der Schaft der Kolbenstange bevorzugt einen kreuzförmigen Querschnitt aufweist. Durch den kreuzförmigen Querschnitt wird die Gefahr des Knickens des Schafts bei Betätigung, insbesondere bei Verwendung der Kolbenstange in Hohlzylindern mit geringen Durchmessern und/oder mit zähflüssigen Wirkstoff-Füllungen zuverlässig ausgeschlossen. Im Anschluß an den Schaft ist auf der dem Wirkstoff Zugewandten Seite eine tellerförmige Stützscheibe angeordnet, die einstückig ineinander übergehend mit der Kolbenstange ausgebildet ist und mit der Innenfläche des Hohlzylinders einen Ringspalt begrenzt. Dadurch, daß die Stützscheibe den Innendurchmesser des Hohlzylinders nicht entlang ihres gesamten Außenumfangs anliegend berührt, ist eine reibungsarme Relativbeweglichkeit der Kolbenstange innerhalb des Hohlzylinders gewährleistet. Die Festlegung des Kolbens an der Kolbenstange durch eine Verschraubung bedingt eine relative Unbeweglichkeit der beiden Teile zueinander in axialer Richtung, was hinsichtlich der exakten Dosierung des zu verabreichenden Wirkstoffs von Vorteil ist. Um eine derartige, relativ unbewegliche Festlegung der beiden Teile aneinander sicherzustellen, ist Voraussetzung, daß das Innengewinde und das Außengewinde ohne axiales Spiel, d.h. mit axialer Vorspannung aneinander festgelegt sind. Eine derartige axiale Vorspannung kann beispielsweise dadurch bewirkt werden, daß das Innengewinde des Kolbens in seiner Bewegungsrichtung kürzer bemessen ist als das Außengewinde der Kolbenstange. Der im wesentlichen topfförmige Kolben wird auf das Außengewinde der Kolbenstange solange aufgeschraubt, bis die Bodenfläche des Kolbens die Stirnfläche der Kolbenstange, die den Abschluß des Außengewindes bildet, unter elastischer Vorspannung anliegend berührt. Der elastomere Werkstoff des Innengewindes ist dadurch gegen die Gewindegänge des Außengewindes der Kolbenstange verspannt, so daß das Gewindespiel aufgehoben ist.

Nach einer anderen Ausgestaltung kann das Innengewinde in Bewegungsrichtung des Kolbens länger bemessen sein, als das Außengewinde. Hierbei ist von Vorteil, daß beim Aufschrauben des topfförmigen Kolbens auf das Außengewinde der Kolbenstange zunächst die Kreisringfläche des Kolbens die Stützscheibe der Kolbenstange anliegend berührt. Wird der Kolben anschließend weiter auf das Außengewinde der Kolbenstange aufgeschraubt, bis der Boden die Stirnfläche anliegend berührt, ergibt sich durch die als Widerlager ausgebildete Stützscheibe eine Stauchung des elastomeren Werkstoffs des Kolbens auf der dem Wirkstoff abgewandten Seite und eine radiale Ausweichbewegung, so daß der gestauchte Werkstoff den Innenumfang des Hohlzylinders dichtend berührt. Neben der Vermeidung von Gewindespiel in axialer Richtung wird durch diese Ausgestaltung eine weiter verbesserte Sicherheit gegen Flüssigkeitsverluste geschaffen.

Gemäß einer anderen Ausgestaltung kann der Kolben mittels eines Außengewindes an einem Innengewinde der Kolbenstange festgelegt sein, wobei das Innengewinde in Bewegungsrichtung des Kolbens kürzer bemessen ist als das Außengewinde des Kolbens. Hierbei ist von Vorteil, daß sich radiale Ausweichbewegungen des Elastomerwerkstoffs des Kolbens beim Befestigen an der Kolbenstange in Richtung des Hohlzylinders nicht einstellen und dadurch hohe Betätigungskräfte und ein Blockieren des Kolbens im Hohlzylinder zuverlässig vermieden werden. Der Kolben wird so lange in die Kolbenstange eingeschraubt, bis sich die Gewindegänge des Außengewindes mit den Gewindegängen des Innengewindes gegeneinander verspannen.

Die Kautschukmischung kann überwiegend aus einem Block-Polymerisat bestehen und einen Gehalt eines Polyolefins aufweisen. Hierbei ist von Vorteil, daß eine derartige Kautschukmischung eine gute elastische Nachgiebigkeit aufweist, daß die Relaxationserscheinungen bei einer erfindungsgemäßen Ausgestaltung und Anwendung vernachlässigbar gering sind und daß eine solche Kautschukmischung in fertigungstechnischer Hinsicht einfach und kostengünstig herstellbar ist. Außerdem ist von Vorteil, daß ein Block-Polymerisat mit einem Gehalt an Polyolefin gegen die gebräuchlichsten Wirkstoffe resistent ist. Als Polyolefin kann Polypropylen zur Anwendung gelangen, wobei der Gehalt bevorzugt 1 bis 30 Gew.% beträgt. In Abhängigkeit von der Höhe des Gehalts des Polypropylens in der Kautschukmischung kann die elastische Nachgiebigkeit des Kolbens an die jeweiligen Gegebenheiten des Anwendungsfalles angepaßt werden. Soll beispielsweise ein vergleichsweise zähflüssiger Wirkstoff verabreicht werden, hat es sich als vorteilhaft bewährt, wenn der Gehalt von Polypropylen 20 bis 30 Gew. % beträgt und der Kolben dadurch die erforderliche Steifigkeit aufweist.

Zur Verbesserung der Gleiteigenschaften des Kolbens im Hohlzylinder kann die Kautschukmischung einen Gehalt von 0,1 bis 2 Gew. % eines Silikongummis enthalten. Durch die verbesserten Gleiteigenschaften werden Stick-Slip-Effekte vermieden. Die Bedienung der Einwegspritze ist vereinfacht und der Wirkstoff kann in genau vorherbestimmten Dosen verabreicht werden.

Die Kautschukmischung kann überwiegend aus einem zumindest teilweise vernetzten Kautschuk und einem thermoplastischen Werkstoff zusammengesetzt sein, wobei die Kautschukmischung beispielsweise aus Styrol-Ethylen-Butylen-Styrol (SEBS) aufgebaut ist.

Die Dichtlippe kann zumindest eine Dichtkante aufweisen, die bevorzugt außenseitig durch zwei einander durchschneidende Kegelflächen begrenzt ist. Bei einer derartigen Ausgestaltung erstreckt sich die Dichtkante im wesentlichen linienförmig und außenumfangsseitig entlang des Kolbens und dichtet die Innenwandung des Hohlzylinders zuverlässig ab. Nach einer davon abweichenden Ausgestaltung kann die Dichtkante beispielsweise außenseitig durch eine Oberfläche mit halbkreisförmigem Querschnitt begrenzt sein. Eine derartige Ausgestaltung ist insbesondere dann von Vorteil, wenn der radiale Anpreßdruck der Dichtlippe an den Hohlzylinder und/oder der Druck innerhalb des Hohlzylinders durch die Verabreichung des Wirkstoffs relativ erhöht ist. Die Dichtlippe ist dadurch gegen abrasiven Verschleiß besser geschützt und dichtet daher auch unter derartigen Bedingungen einwandfrei ab.

Der Kolben kann radial innerhalb der Dichtlippe mit einer Aussparung versehen sein. Die Aussparung ist bevorzugt durch eine in die Stirnfläche des Kolbens eingreifende, umlaufende Nut gebildet. Bei Bewegung der Kolbenstange in Richtung der Injektionsnadel und damit verbundener Verabreichung des Wirkstoffs, baut sich innerhalb der kreisringförmig umlaufenden Nut ein im Vergleich zur Atmosphäre relativer Überdruck auf, der die konstruktions- und werkstoffbedingte Anpressung der Dichtlippe an die Innenwand des Hohlzylinders zusätzlich unterstützt. In Abhängigkeit von der Viskosität des Wirkstoffs und dem Druck, der sich dadurch bei Verabreichung des Wirkstoffs innerhalb des Hohlzylinders aufbaut, paßt sich die Anpreßkraft der Dichtlippe an den Hohlzylinder selbsttätig an.

Der Kolben kann radial innerhalb der Nut eine Stirnfläche aufweisen, die eine an die Stirnwand des Hohlzylinders angepaßte Gestalt aufweist. Durch diese Ausgestaltung wird bewirkt, daß der Totraum innerhalb des Hohlzylinders bei vollständig eingefahrener Kolbenstange auf ein Minimum beschränkt ist. Die Stirnfläche des elastomeren Kolbens wird bei Betätigung der Einwegspritze fast bis an die Austrittsöffnung des Hohlzylinders bewegt und verdrängt den im Hohlzylinder vorhandenen Wirkstoff nahezu vollständig.

Die Stirnfläche begrenzt bevorzugt einen Vorsprung, der die Dichtlippe in Richtung der Stirnwand überragt. Dadurch können Flüssigkeitsbestandteile des Wirkstoffs, die sich im verengten Austrittsbereich des Hohlzylinders befinden, nahezu vollständig verabreicht werden.

Der durch das überragende Ende gebildete Überstand des Vorsprungs kann in einer vorteilhaften Ausgestaltung so bemessen sein, daß die sich bei einer Anpressung des Kolbens an die Stirnwand ergebende radiale Verlagerung der Dichtlippe die Nut im wesentlichen ausfüllt. Die Flüssigkeitsbestandteile des Wirkstoffs, die sich während der Bewegung der Kolbenstange in Richtung der Injektionsnadel innerhalb der Nut befinden, werden bei einer Anschlagberührung des Kolbens an der Stirnwand des Hohlzylinders durch die elastische Verformung des Kolbens aus der Nut in Richtung der Injektionsnadel verlagert. Dabei ist sichergestellt, daß sich trotz der Verformung des Kolbens keine Flüssigkeitsbestandteile des Wirkstoffs an der Dichtlippe vorbei in Richtung der Atmosphäre verlagern.

Die Stirnfläche kann durch zumindest eine in radialer Richtung verlaufende Ausnehmung unterbrochen sein, die sich vom Außenumfang zur Mitte erstreckt. Dadurch wird gewährleistet, daß der Wirkstoff bei Anschlagberührung der Stirnseite des Kolbens an der Stirnwand des Hohlzylinders nicht in Hohlräume eingeschlossen wird, sondern daß stets eine flüssigkeitsleitende Verbindung in Richtung der Injektionsnadel besteht.

Ein Ausführungsbeispiel der erfindungsgemäßen Einwegspritze wird nachfolgend anhand der Zeichnungen näher erläutert.

In Fig. 1 ist die erfindungsgemäße Einwegspritze mit dem Wirkstoff gefüllt.

In Fig. 2 ist die Einwegspritze aus Fig. 1 entleert.

In Fig. 3 ist ein Ausschnitt aus der Einwegspritze gezeigt, wobei der Wirkstoff noch nicht vollständig entleert ist.

In Fig. 4 ist die Einzelheit aus Fig. 3 gezeigt, wobei die Kolbenstange in Richtung der Injektionsnadel relativ weiter verschoben wurde und der Wirkstoff am weitestgehenden entleert ist.

In den Fig. 5 und 6 ist ein weiteres Ausführungsbeispiel gezeigt, wobei der Kolben durch ein Außengewinde in einem Innengewinde der Kolbenstange befestigt ist.

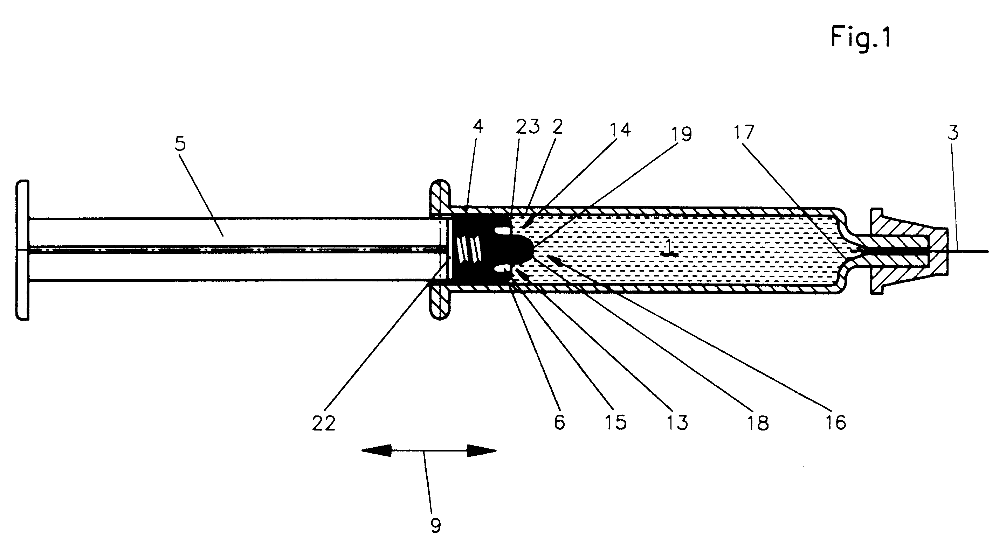

In Fig. 1 ist ein Ausführungsbeispiel einer Einwegspritze gezeigt, die mit einem flüssigen Wirkstoff 1 gefüllt ist. Die Einwegspritze umfaßt einen Hohlzylinder 2, dessen eines axiales Ende von einem auf eine Kolbenstange 5 aufgesetzten Kolben 4 flüssigkeitsdicht verschlossen ist und dessen axiales anderes Ende mit einer Injektionsnadel 3 versehen ist. Der Kolben 4 besteht aus einem elastisch nachgiebigen, thermoplastisch verarbeitbaren Kautschukmaterial, bevorzugt TPE, und dichtet den Wirkstoff innerhalb des Hohlzylinders gegen die Umgebung ab. Der Kolben 4 ist topfförmig ausgebildet und weist innerhalb seiner Ausnehmung eine Innengewinde 7 auf, mit dem er auf einem Außengewinde 8 der Kolbenstange 5 befestigt ist. Der Kolben 4 ist unter axialer Vorspannung auf der Kolbenstange festgelegt, so daß ein Gewindespiel dadurch ausgeglichen ist. Im hier dargestellten Ausführungsbeispiel liegt der kreisringförmige Rand des Kolbens 4 an der Stützscheibe 22 der Kolbenstange 5 unter elastischer Vorspannung dichtend an, wobei sich durch die Stauchung des elastomeren Werkstoffs eine radiale Aufweitung und zusätzliche Abdichtung gegenüber der Innenwand des Hohlzylinders 2 einstellt.

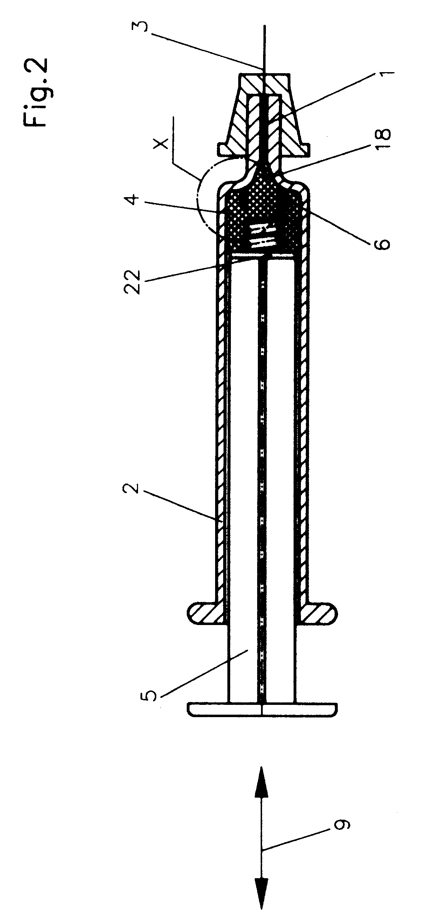

In Fig. 2 ist die erfindungsgemäße Einwegspritze im entleerten Zustand gezeigt. Die dem Wirkstoff 1 zugewandte Stirnfläche 14 des Kolbens 4 berührt die innenseitige Stirnwand 17 des Hohlzylinders 2 anliegend, wobei der Vorsprung 18 die Auslaßöffnung des Hohlzylinders 2 zumindest teilweise ausfüllt.

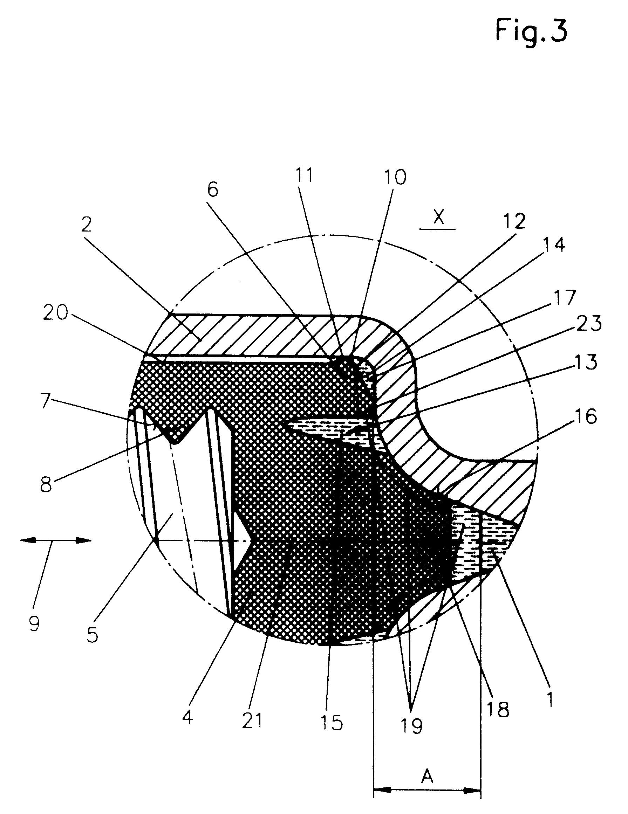

In Fig. 3 ist die Einwegspritze im Bereich ihrer Austrittsöffnung dargestellt, wobei der Wirkstoff 1 noch nicht vollständig entleert ist. Der Kolben 4 berührt die Innenumfangswand des Hohlzylinders 2 mit seiner Dichtlippe 6 unter radialer Vorspannung dichtend, wobei die Dichtlippe 6 in diesem Ausführungsbeispiel eine Dichtkante 10 umfaßt. Der Kolben 4 berührt die Innenwandung des Hohlzylinders 2 radial außerhalb der Nut 15 außerdem mit der Dichtnase 23 und radial innerhalb der Nut 15 mit der Stirnfläche 16. Der Vorsprung 18 ist in diesem Betriebszustand bereits durch die Austrittsöffnung des Hohlzylinders 2 in seiner Position zentriert, wobei zur Förderung des Wirkstoffs 1 in Richtung der Injektionsnadel 3 Ausnehmungen 19 vorgesehen sind, wobei zur Kanalisierung des Wirkstoffs 1 in Richtung der Injektionsnadel 3 Ausnehmungen 19 vorgesehen sind, die sich innerhalb des Vorsprungs 18 in Strömungsrichtung erstrecken.

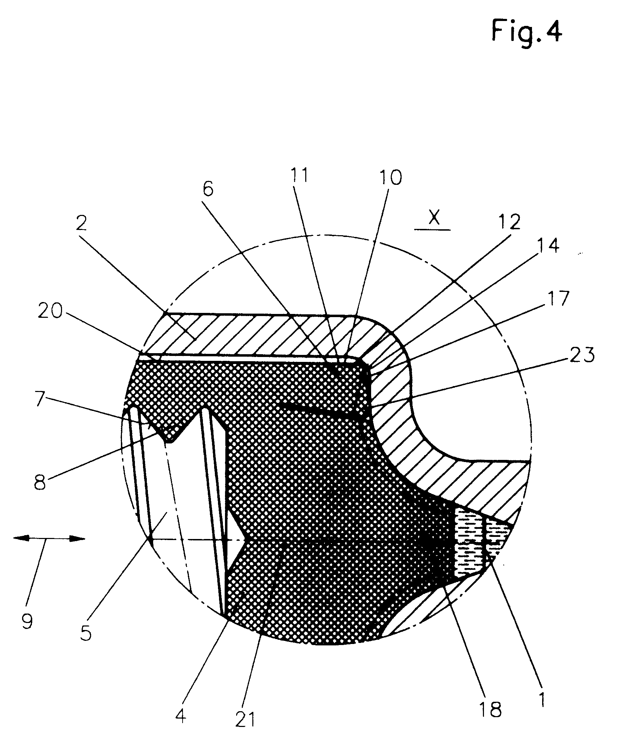

In Fig. 4 ist die Einzelheit aus Fig. 3 dargestellt, wenn die Kolbenstange 5 mit dem daran befestigten Kolben 4 weiter in Richtung der Austrittsöffnung des Hohlzylinders 2 bewegt wird. Ausgehend von der Darstellung in Fig. 3 hat sich der Teilbereich des Kolbens radial außerhalb der Nut 15 durch die Formgebung der Innenwandung des Hohlzylinders 2 radial nach innen verlagert, wobei die Dichtkante 10 der Dichtlippe 6 den Hohlzylinder 2 nach wie vor dichtend berührt. Die Dichtnase 23 bedingt eine Führung der Dichtlippe 6, wobei durch die radiale Verlagerung die Nut 15 im wesentlichen verschlossen und die darin befindlichen Flüssigkeitsbestandteile des Wirkstoffs 1 durch die Ausnehmungen 19 des Vorsprungs 18 in Richtung der Injektionsnadel 3 verdrängt sind. Der Kolben 4 schließt in diesem Betriebszustand, wenn alle Flüssigkeitsbestandteile des Wirkstoffs 1 aus der Nut 15 verdrängt sind, die Austrittsöffnung des Hohlzylinders 2 vollständig ab. Die Größe des Überstands A des Vorsprungs 18 ist im wesentlichen abhängig von der Form des Hohlzylinders 2 im Bereich seiner Austrittsöffnung. Der Überstand A muß in Verbindung mit der geometrischen Ausgestaltung des Vorsprungs 19 derart bemessen sein, daß die Stirnfläche 14 des Kolbens 4 die Austrittsöffnung des Hohlzylinders 2 erst dann vollständig abschließt, wenn sich radial außerhalb und in der Nut 15 kein Wirkstoff 1 mehr befindet.

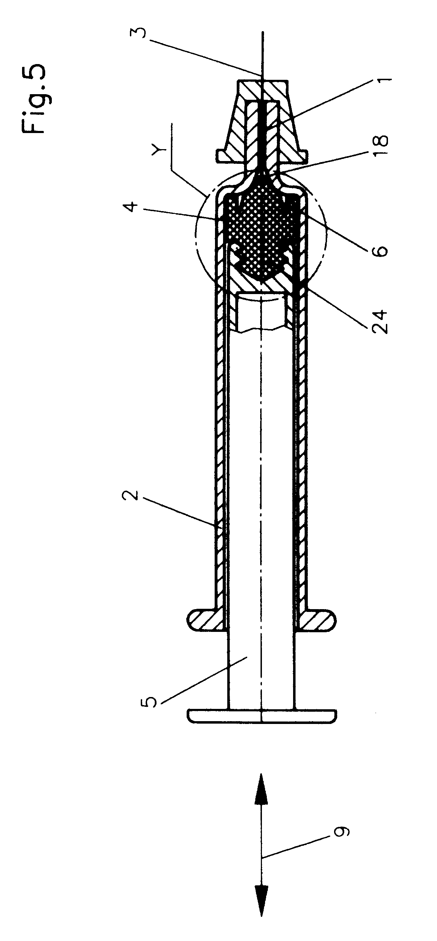

In Fig. 5 ist ein weiteres Ausführungsbeispiel der erfindungsgemäßen Einwegspritze gezeigt, wobei der Wirkstoff 1 aus dem Hohlzylinder 2 weitgehend entleert ist. Der Kolben 4 weist auf der dem Wirkstoff 1 abgewandten Seite ein Außengewinde 24 auf, das einstückig angeformt ist, wobei das Außengewinde 24 mit einem Innengewinde 25 der Kolbenstange 5 verbunden ist. Die dem Wirkstoff 1 zugewandte Stirnfläche 14 des Kolbens unterscheidet sich von dem Ausführungsbeispiel aus Fig. 3 nicht.

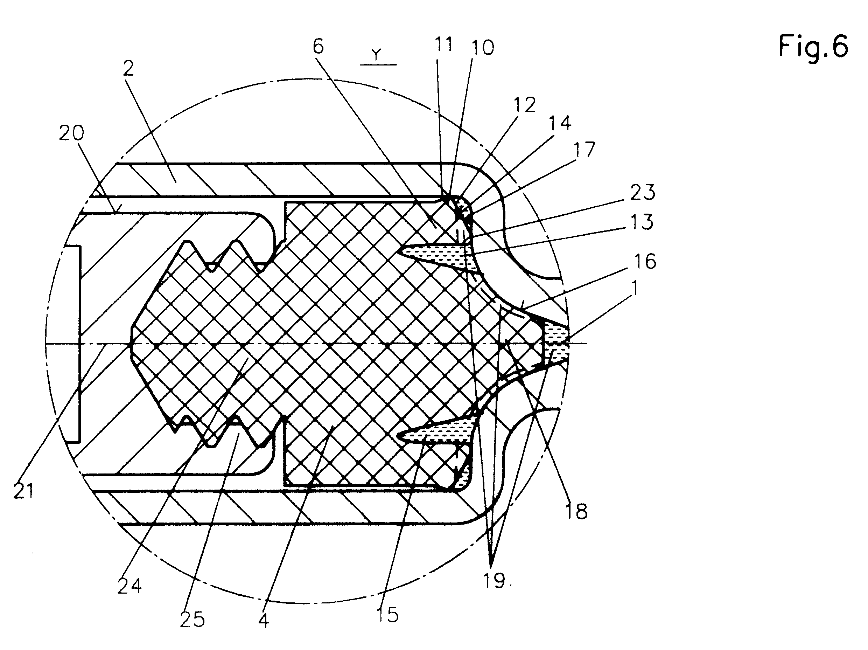

In Fig. 6 ist ein Ausschnitt aus der Einwegspritze aus Fig. 5 in vergrößertem Maßstab dargestellt. Es ist zu erkennen, daß die Außenumfangsfläche des Kolbens 4 im Anschluß an die Montage nicht gestaucht und daher in radialer Richtung nicht aufgeweitet wird. Hierbei ist von Vorteil, daß der Kolben 4 außenumfangsseitig die Innenwandung des Hohlzylinders nur mit der Dichtkante 10 der Dichtlippe 6 berührt, wodurch die Betätigungskräfte beim Verabreichen einer Injektion auf ein Minimum reduziert sind.

## Patentansprüche

1. Einwegspritze zur Verabreichung flüssiger Wirkstoffe an lebende Menschen oder Tiere, umfassend einen mit dem Wirkstoff gefüllten Hohlzylinder, auf den eine Injektionsnadel aufsetzbar ist und der einen Kolben umschließt, der mittels einer Kolbenstange in Richtung der Injektionsnadeln bewegbar ist, dadurch gekennzeichnet, daß der Kolben (4) aus einer themoplastisch verarbeitbaren Kautschukmischung erzeugt und in Richtung des Wirkstoffes (1) durch eine den Hohlzylinder (2) berührende, umlaufende und in radialer Richtung einfederbare Dichtlippe (6) begrenzt ist.

2. Einwegspritze nach Anspruch 1, dadurch gekennzeichnet, daß der Kolben (4) mittels eines Innengewindes (7) an einem Außengewinde (8) der Kolbenstange (5) festgelegt ist und daß das Innengewinde (7) in Bewegungsrichtung (9) des Kolbens (4) kürzer bemessen ist als das Außengewinde (8).

3. Einwegspritze nach Anspruch 1, dadurch gekennzeichnet, daß der Kolben (4) mittels eines Außengewindes (24) an einem Innengewinde (25) der Kolbenstange (5) festgelegt ist und daß das Innengewinde (25) in Bewegungsrichtung (9) des Kolbens (4) kürzer bemessen ist als das Außengewinde (24) des Kolbens (4).

4. Einwegspritze nach Anspruch 1 bis 3, dadurch gekennzeichnet, daß die Kautschukmischung überwiegend aus einem Blockpolymerisat besteht.

5. Einwegspritze nach Anspruch 4, dadurch gekennzeichnet, daß die Kautschukmischung einen Gehalt eines Polyolefins aufweist.

6. Einwegspritze nach Anspruch 5, dadurch gekennzeichnet, daß als Polyolefin Polypropylen zur Anwendung gelangt und daß der Gehalt 1 bis 30 Gew. % beträgt.

7. Einwegspritze nach Anspruch 4 bis 6, dadurch gekennzeichnet, daß die Kautschukmischung einen Gehalt von 0,1 bis 2 Gew. % eines Silikongummis enthält.

8. Einwegspritze nach Anspruch 1 bis 7, dadurch gekennzeichnet, daß die Dichtlippe (6) zumindest eine Dichtkante (10) aufweist, die außenseitig durch zwei einander durchschneidende Kegelflächen (11, 12) begrenzt ist.

9. Einwegspritze nach Anspruch 1 bis 8, dadurch gekennzeichnet, daß der Kolben (4) radial innerhalb der Dichtlippe (6) mit einer Aussparung (13) versehen ist.

10. Einwegspritze nach Anspruch 9, dadurch gekennzeichnet, daß die Aussparung (13) durch eine in die Stirnfläche (14) des Kolbens (4) eingreifende, umlaufende Nut (15) gebildet ist.

11. Einwegspritze nach Anspruch 10, dadurch gekennzeichnet, daß der Kolben (4) radial innerhalb der Nut (15) eine Stirnfläche (16) aufweist, die eine an die Stirnwand (17) des Hohlzylinders (2) angepaßte Gestalt aufweist.

12. Einwegspritze nach Anspruch 9 bis 11, dadurch gekennzeichnet, daß die Stirnfläche (14) einen Vorsprung (18) begrenzt, der die Dichtlippe (6) in Richtung der Stirnwand (17) überragt.

13. Einwegspritze nach Anspruch 12, dadurch gekennzeichnet, daß der durch das überragende Ende gebildete Überstand (A) des Vorsprungs (18) so bemessen ist, daß die sich bei einer Anpressung des Kolbens (4) an die Stirnwand (17) ergebende radiale Verlagerung der Dichtlippe (6) die Nut (15) im wesentlichen ausfüllt.

14. Einwegspritze nach Anspruch 11 bis 13, dadurch gekennzeichnet, daß die Stirnfläche (16) durch zumindest eine in radialer Richtung verlaufende Ausnehmung (19) unterbrochen ist, die sich vom Außenumfang (20) bis zur Mitte (21) erstreckt.
